# EUROPÄISCHE PATENTSCHRIFT

(11) **EP 1 844 764 B1**
(45) Veröffentlichungstag und Bekanntmachung des Hinweises auf die Patenterteilung: **10.05.2017**
(21) Anmeldenummer: 07000329.8
(22) Anmeldetag: 09.01.2007
(51) Int. Cl.: A61K 9/00, A61K 9/08, A61K 47/10, A61K 36/00, A61K 47/26, A61K 47/36

(54) **Zusammensetzung zur Verwendung als Laxativum**
Compound for use as a laxative
Composition destinée à l'utilisation en tant que laxatif

(30) Priorität: 12.04.2006 DE 102006017672
(43) Veröffentlichungstag der Anmeldung: 17.10.2007
(73) Patentinhaber: Maria Clementine Martin Klosterfrau Vertriebsgesellschaft mbH, 50670 Köln (DE)
(72) Erfinder: Vestweber, Anna-Maria, Dr. med., 51399 Burscheid (DE); Greve, Harald, Dr., 40545 Düsseldorf (DE)
(74) Vertreter: Von Rohr Patentanwälte Partnerschaft mbB

(56) Entgegenhaltungen:
- WO-A1-97/03685
- WO-A1-2005/007170
- US-A1- 2005 142 099

## Beschreibung

Die vorliegende Erfindung betrifft eine Zusammensetzung, insbesondere pharmazeutische Zusammensetzung, vorzugsweise zur Verwendung als Laxativum (Abführmittel, Laxans). Insbesondere betrifft die vorliegende Erfindung eine Zusammensetzung, insbesondere pharmazeutische Zusammensetzung, auf Basis mindestens eines Polyols sowie mindestens eines Ballaststoffes und/oder mindestens eines Präbiotikums und die Verwendung dieser Zusammensetzung als Laxativum (Abführmittel, Laxans).

Die Ursache für Obstipationen können vielfältig sein: Oftmals sind eine zu geringe Flüssigkeitszufuhr, eine unausgewogene Ernährung mit zu wenig Ballaststoffen und/oder fehlende körperliche Bewegung Auslöser für eine Obstipation. Aber auch Streß, Arzneimittel oder eine gestörte Darmflora können dazu beitragen, daß der Darm träge wird. Dem Darminhalt wird zuviel Wasser entzogen, so daß er trockener, fester und weniger gleitfähig wird und infolgedessen die Darmentleerung mühsam und oft auch schmerzhaft wird.

Zur Behandlung von Obstipationen werden sogenannte Laxativa verabreicht, die in vielfältigster Form eingesetzt werden können. Laxativa - synonym auch als "Laxantien" oder "Abführmittel" bezeichnet - gibt es in den vielfältigsten Erscheinungsformen, hierunter auch zahlreiche pflanzliche Abführmittel. Im allgemeinen bezeichnet man unter diesem Begriff Mittel zur Beseitigung der Stuhlverstopfung ("Obstipation") durch Ingangsetzen der Stuhlentleerung (Defäkation) und/oder durch Aufweichen des eingedickten Stuhles (Fäzes).

Als Laxantien unterschieden werden zum einen Gleitmittel (Lubricantia), wie schlecht- oder nichtresorbierbare Öle, z. B. Glycerin und Paraffin, Mineralöle mit Zusatz von Agar-Agar als Füllmittel bzw. Phenolphthalein als dickdarmwirksames diphenolisches Abführmittel, wobei bei chronischer Anwendung dieser Öle unter anderem die Gefahr von Vitaminmangelzuständen (z. B. Vitamine A, D, E und K) besteht, und zum anderen Füllmittel, d. h. unverdauliche und nichtresorbierbare, unter Wasseraufnahme quellende und durch Volumenzunahme die Auslösung des Defäkationsreflexes begünstigende Mittel, wie Agar-Agar, Methylcellulose und als Hausmittel z. B. Leinsamen. Darüber hinaus dienen als Abführmittel Stoffe mit Hemmeffekt gegenüber Flüssigkeits- und Natriumresorption bei gleichzeitiger Begünstigung der Flüssigkeitsabsonderung (samt Elektrolyten, wie Na⁺, K⁺, Ca²⁺, Cl⁻ etc.) im Dickdarmbereich, d. h. antiadsorptiv und sekretagog wirkende Abführmittel (sogenannte Dickdarmmittel) bekannt, und zwar insbesondere Anthrachinone, die wirksam sind durch darmbakteriell entstandene Endprodukte ihrer Aglykone, sowie diphenolische Abführmittel, wie Phenolphthalein, Bisacodyl und dessen Schwefelsäureester sowie Natriumpicosulfat, welche durch im Dickdarm frei werdende Diphenole wirken, sowie Gallensäuren. Weiterhin sind osmotische Abführmittel ("Osmolaxantien") bekannt, welche durch Zurückhalten osmotisch äquivalenter Flüssigkeitsmengen in der Darmlichtung (Darmlumen) wirken, so z. B. Lävulose, sowie, als salinische Abführmittel, Sulfationen (wie z. B. Karlsbader Salz, Magnesiumsulfatwässer, Glaubersalz etc.). Schließlich kommen auch Laxantien mit Stimulation der Prostaglandinsynthese im Dünndarm, wie z. B. Rizinusöl, zum Einsatz.

Alle vorgenannten Abführmittel sind bei chronischer Verabfolgung durch die Gefahr von Wasser-/Elektrolythaushalt-Störungen und Dickdarmveränderungen belastet, manche auch - substanzspezifisch - durch besondere Nebenwirkungen.

Weiterhin können mit den Laxantien des Standes der Technik oftmals schwerwiegende Nebenwirkungen verbunden sein, insbesondere bei längerer und hochdosierter Anwendung. Diese Nebenwirkungen umfassen insbesondere Elektrolytverluste (unter anderem Kaliumverlust und dadurch Verstärkung der Obstipation), Melanosis coli (z. B. bei Abführmitteln auf Basis von Anthrachinonderivaten), Bildung von Fremdkörpergranulomen (z. B. bei Paraffinölen), hämorrhagische Enteritiden und lebensbedrohliche Überempfindlichkeitsreaktionen (z. B. bei Phenolphthalein) sowie akute Blähungen (z. B. bei Quellstoffen) oder Bauchschmerzen (z. B. bei Anthrachinonderivaten).

Laxantienabusus, d. h. die mißbräuchliche Einnahme von Abführmitteln, oft psychisch bedingt, manifestiert sich klinisch in Kaliurese, intermittierender Hypokaliämie und Atrophie der Darmmukosa. Durch Kaliumverlust können hypokaliämische Lähmungen auftreten.

Daneben sind auch sogenannte natürliche Abführmittel bekannt, insbesondere in Form von sogenannten natürlichen Laxantienkombinationen, wie sie in bestimmten pflanzlichen Stoffen enthalten sind, z. B. Extractum Colocynthidis ("Koloquinten-Extrakt"), Aloe, Jalapa-Harz, Podophyllin (welches aber als potentiell terato- und karzinogen gilt) etc.

Für weitergehende Einzelheiten zu Laxativa kann beispielsweise verwiesen werden auf Pschyrembel, Medizinisches Wörterbuch, 257. Auflage, Verlag Walter de Gruyter, 1993, Seite 852, Stichwort: "Laxanzien", sowie auf Roche Lexikon Medizin, 3. Auflage, Verlag Urban & Schwarzenberg, 1993, Seite 3, Stichwort: "Abführmittel", deren gesamter diesbezüglicher Offenbarungsgehalt hiermit durch Bezugnahme eingeschlossen ist.

Weiterhin sind natürliche Abführmittel auf Basis von Flohsamen (*Psyllium*) oder deren Bestandteilen und dergleichen bekannt, welche aber oftmals keine ausreichende abführende Wirkung liefern.

Speziell als Osmolaxans sind Präparate auf der Basis von Macrogol als Wirkstoff bekannt. Derartige Handelspräparate liefern aber nicht immer die gewünschte abführende Wirkung, insbesondere fehlt es oftmals an einer Stimulation der Darmtätigkeit. WO97/03685, WO2005/007170, US2005/142099 legen eine Kombination von Polyol (Polyethylenglycol) und einen Ballaststoff bzw. Lactulose (ein Präbiotikum) für ein Laxativum offen. Somit liegt der vorliegenden Erfindung das Problem zugrunde, eine Zusammensetzung, insbesondere pharmazeutische Zusammensetzung, bereitzustellen, welche sich zur Anwendung als Laxativum eignet und die zuvor geschilderten Nachteile des Standes der Technik zumindest weitgehend vermeidet oder aber wenigstens abschwächt.

Gegenstand der vorliegenden Erfindung - gemäß einem **ersten** Aspekt der vorliegenden Erfindung - ist somit eine Zusammensetzung, insbesondere pharmazeutische Zusammensetzung, wie sie im Anspruch 1 offenbart ist.

Der Begriff "pharmazeutische Zusammensetzung" bzw. "pharmazeutische Zubereitung" oder dergleichen, wie er im Rahmen der vorliegenden Erfindung verwendet wird, ist sehr umfassend zu verstehen und bezeichnet nicht nur pharmazeutische Präparate bzw. Pharmazeutika als solche, sondern auch sogenannte Medizinprodukte, Nahrungsergänzungsmittel oder dergleichen.

Bei allen nachstehend genannten Mengenangaben ist zu beachten, daß diese im Rahmen der erfindungsgemäßen Zusammensetzung bzw. Kombination vom Fachmann derart auszuwählen sind, daß sie sich in Summe stets zu 100 Gew.-% ergänzen. Dies versteht sich für den Fachmann aber von selbst.

Die Menge an Polyol(en), die in der erfindungsgemäßen Zusammensetzung vorhanden ist bzw. sind, kann in weiten Bereichen variieren. Im allgemeinen enthält die Zusammensetzung nach der vorliegenden Erfindung Polyol(e), bezogen auf die Zusammensetzung, in Mengen von 10 bis 99,5 Gew.-%, insbesondere 40 bis 99 Gew.-%, vorzugsweise 60 bis 99 Gew.-%, besonders bevorzugt 80 bis 98 Gew.-%, ganz besonders bevorzugt 90 bis 97 Gew.-%. Dennoch kann es einzelfallbedingt oder anwendungsbezogen erforderlich sein, von den vorgenannten Mengen abzuweichen, ohne daß der Rahmen der vorliegenden Erfindung verlassen ist.

Was die Menge an Ballaststoff(en) und/oder Präbiotikum bzw. Präbiotika anbelangt, so kann diese gleichermaßen in weiten Bereichen variieren. Im allgemeinen enthält die erfindungsgemäße Zusammensetzung, bezogen auf die Zusammensetzung, Ballaststoff(e) und/oder Präbiotikum bzw. Präbiotika in Mengen von 0,5 bis 30 Gew.-%, insbesondere 0,6 bis 10 Gew.-%, vorzugsweise 0,7 bis 5 Gew.-%, besonders bevorzugt 0,8 bis 3 Gew.-%, ganz besonders bevorzugt 1 bis 2 Gew.-%.

Was das erfindungsgemäß eingesetzte Polyol oder die Mischung der Polyole anbelangt, so ist bzw. sind diese(s) im allgemeinen osmotisch wirksam, insbesondere durch osmotische Bindung von Wasser. Ferner können die erfindungsgemäß eingesetzten Polyole in einer Flüssigkeit, insbesondere Wasser, löslich und/oder quellfähig sein. Dabei sollte das erfindungsgemäß eingesetzte Polyol zumindest im wesentlichen nichtresorbierbar sein, d. h. im Magen/Darm-Trakt nach seiner peroralen Applikation nichtresorbiert werden. Wie zuvor beschrieben, ist das erfindungsgemäß eingesetzte Polyol vorteilhafterweise osmotisch wirksam, insbesondere bindet es osmotisch Wasser. Bei peroraler Applikation der erfindungsgemäßen Zusammensetzung, im allgemeinen mit Wasser, nimmt das erfindungsgemäß eingesetzte Polyol die applizierte Menge an Wasser, sozusagen wie ein unsichtbarer Schwamm, auf und transportiert es durch den Magen/Darm-Trakt in den Dickdarm, ohne daß dem Körper Flüssigkeit entzogen wird. Der verhärtete Stuhl wird auf diese Weise aufgeweicht und ein leichtes Ausscheiden wird ermöglicht. Gleichzeitig führt, wie nachfolgend noch geschildert, der in Kombination hiermit applizierte Ballaststoff bzw. das in Kombination hiermit applizierte Präbiotikum zu einer Anregung der Darmtätigkeit. Insgesamt wird also dafür gesorgt, daß ein normaler, weicher und leichter Stuhl auf besonders schonende und zuverlässige Weise erreicht und die Darmtätigkeit angeregt wird.

Im allgemeinen wird als Polyol erfindungsgemäß ein Polyalkylenglykol, vorzugsweise eine Polyethylenglykol, eingesetzt.

Das Molekulargewicht (Molekularmasse) des eingesetzten Polyols, insbesondere Polyalkylenglykols, vorzugsweise Polyethylenglykols, kann in weiten Bereichen variieren. Im allgemeinen liegt das mittlere Molekulargewicht des eingesetzten Polyols, insbesondere Polyalkylenglykols, vorzugsweise Polyethylenglykols, im Bereich von 1.000 bis 10.000 g/mol (Dalton), insbesondere 2.000 bis 8.000 g/mol, vorzugsweise 2.500 bis 5.500 g/mol, bevorzugt 3.000 bis 5.000 g/mol, besonders bevorzugt 3.200 bis 4.200 g/mol.

In erfindungsgemäß bevorzugter Weise wird als Polyol ein Polyethylenglykol mit einem mittleren Molekulargewicht im Bereich von 2.500 bis 5.000 g/mol, bevorzugt 3.000 bis 5.000 g/mol, ganz besonders bevorzugt 3.200 bis 4.200 g/mol.

In erfindungsgemäß ganz bevorzugter Weise kann als Polyol ein Polyethylenglykol mit einem mittleren Molekulargewicht von etwa 3.350 g/mol oder etwa 4.000 g/mol oder eine Mischung eines Polyethylenglykols mit einem mittleren Molekulargewicht von etwa 3.350 g/mol und eines Polyethylenglykols mit einem mittleren Molekulargewicht von etwa 4.000 g/mol, vorzugsweise vom Typ Macrogol 3350 und/oder Macrogol 4000, eingesetzt werden.

Macrogol besteht aus einer osmotisch wirksamen, nichtresorbierbaren Struktur mit einem mittleren Molekulargewicht von 3.350 g/mol oder 4.000 g/mol und wird im Rahmen der erfindungsgemäßen Kombination bzw. Zusammensetzung zur Behandlung der Obstipation verwendet. Macrogol, sowohl mit einem Molekulargewicht von 3.350 g/mol als auch mit einem Molekulargewicht von 4.000 g/mol, liegt in Form eines Pulvers oder Granulats vor und kann - zusammen mit dem Ballaststoff bzw. Präbiotikum - im Wasser aufgelöst bzw. suspendiert und als Lösung oder Suspension mit Wasser (z. B. ca. 125 ml pro 10 bis 50 g der Zusammensetzung) getrunken werden. Die Lösung bzw. Suspension passiert dann ohne weitere physikalische und pharmakologische Effekte den Magen und oberen Darmtrakt. Erst im Dickdarm kommt dann der zuvor geschilderte, gewünschte osmotische Effekt in vollem Umfang physikalisch zum Tragen: Wasser wird aufgrund eines osmotischen Vorgangs in den Dickdarm zurückgeholt und bindet sich an das Macrogol bzw. an das Polyol, welches dieses Wasser bindet. Auf diese Weise kommt es zu einer Erweichung des Stuhls und einer Zunahme der Stuhlmenge, wodurch es durch Überdehnungsreflexe im Colon zu einer Peristaltikanregung kommt, die im Rektumbereich zum Stuhlreflex führt. Das Polyol bzw. das Macrogol wird bei diesem Vorgang nicht aus dem Darm im Körper aufgenommen, sondern unverändert ausgeschieden.

Auch bei einer regelmäßigen Einnahme besteht nicht die Gefahr der Gewöhnung, die man von herkömmlichen Abführmitteln kennt, deren Wirkung auch nicht über physikalische Effekte erklärt werden kann. Der Haupteffekt der erfindungsgemäßen Zusammensetzung ist vielmehr die Wasserbindung im Darmvolumen durch Osmose, so daß es zum Dehnungsreflex im Colon und zum Auslösen der Darmperistaltik mit dem Erfolg der erleichterten Stuhlentleerung kommt, gepaart mit der Anregung der natürlichen Darmtätigkeit durch die Kombination mit dem Ballaststoff und/oder dem Präbiotikum.

Was die erfindungsgemäß eingesetzten Ballaststoffe und/oder Präbiotika anbelangt, so können diese insbesondere ausgewählt sein aus der Gruppe von Cellulose, Lignin, Pentosanen, Keratinen, Agar-Agar, löslichen Ballaststoffen, wie Fructo- oder Galactooligosacchariden, vorzugsweise Inulin, sowie deren Derivaten und Mischungen.

Der Begriff der Ballaststoffe, wie er im Rahmen der vorliegenden Erfindung verwendet wird, bezeichnet für den menschlichen Körper unverdauliche Nahrungsbestandteile, wie insbesondere die vorgenannten Ballaststoffe, d. h. Cellulose, Lignin, Keratine, Pentosane, Agar-Agar etc. Wichtigster Ballaststoff ist die Cellulose, die neben Lignin und Pentosanen in pflanzlichen Zellwänden vorkommt, die ihrerseits häufiger Bestandteil von pflanzlichen Fasern sind (daher auch die Bezeichnung Faserstoffe, mit der in diesem Zusammenhang die Ballaststoffe gemeint sind). Diese polymeren Ballaststoffe können nicht im menschlichen Verdauungssystem enzymatisch in Oligo- oder Monomere gespalten und als solche resorbiert werden; sie passieren vielmehr das Verdauungssystem weitgehend unverändert. Als sogenannte lösliche Ballaststoffe bezeichnet man Verbindungen wie Fructo- oder Galactooligosaccharide (z. B. Inulin), welche die Herstellung klarer ballaststoffhaltiger Getränke ermöglichen. Vermutlich aufgrund mechanischer Reizung der Darmwände und ihrer Eigenschaft, schädliche Stoffwechselprodukte zu binden, sind Ballaststoffe für die Verdauung wichtig und regen die Darmperistaltik an. Für weitere diesbezügliche Einzelheiten zu dem Begriff der Ballaststoffe kann beispielsweise verwiesen werden auf Römpp Chemielexikon, Georg Thieme Verlag Stuttgart/New York, 1996, 10. Auflage, Band 1, Seiten 351/352, Stichwort: "Ballaststoffe", sowie die dort referierten Literaturstellen, wobei der gesamte Offenbarungsgehalt der vorgenannten Literaturstellen hiermit durch Bezugnahme eingeschlossen ist.

Was den Begriff des Präbiotikums anbelangt, wie er im Rahmen der vorliegenden Erfindung verwendet wird, so bezeichnet dieser insbesondere unverdauliche Nahrungsbestandteile mit positiven Auswirkungen auf die Gesundheit. Präbiotika stimulieren selektiv das Wachstum bestimmter Darmbakterien.

In erfindungsgemäß bevorzugter Weise wird als Präbiotikum Inulin - synonym auch als Dahlin, Alantin oder Alant-Stärke bezeichnet - eingesetzt; hierbei handelt es sich um ein farbloses hygroskopisches Pulver, das sich in Wasser, insbesondere beim Erwärmen, zu einer kolloidalen Lösung auflöst, aber unlöslich in Alkoholen und Ethern ist. Inulin ist ein lineares Polyfructosan mit 30 bis 60 Fructoseeinheiten in β2-1-Bindung, die in der furanosiden Form vorliegen. Das Molekulargewicht liegt bei ca. 5.000 g/mol. Inulin findet sich allein oder mit Stärke zusammen als Reservekohlenhydrat in Dahlienknollen, Artischocken, Topinamburgknollen, Zichorienwurzeln, Löwenzahnwurzeln, in den Zellen von Alant und anderen Korbblütlern, seltener auch in verwandten Pflanzenfamilien (Campanulaceae, Lobeliaceae).

Inulin ist als Präbiotikum besonders geeignet, da es einerseits selektiv das Wachstum gesunder Darmbakterien anregt und zudem als löslicher Ballaststoff diabetikergeeignet ist und nichtkariogene Eigenschaften besitzt. Insbesondere kann das Inulin zur Ballaststoffanreicherung verwendet werden.

Für weitere Einzelheiten zu der Substanz Inulin kann beispielsweise verwiesen werden auf Römpp Chemielexikon, Georg Thieme Verlag Stuttgart/New York, 1997, Band 3, Seiten 1948/1949, Stichwort: "Inulin", sowie die dort referierte Literatur, wobei der gesamte Offenbarungsgehalt der vorgenannten Literaturstellen hiermit durch Bezugnahme eingeschlossen ist.

In erfindungsgemäß bevorzugter Weise kann der Ballaststoff und/oder das Präbiotikum in Form von Inulin, Flohsamen, Leinsamen, Guar und/oder Lactulose zugesetzt sein. Erfindungsgemäß ganz besonders bevorzugt ist - wie zuvor beschrieben - die Verwendung von Inulin als Ballaststoff und/oder Präbiotikum.

Neben den vorgenannten Inhaltsstoffen (d. h. Polyol(e) einerseits und Ballaststoff(e) und/oder Präbiotikum bzw. Präbiotika andererseits) kann die erfindungsgemäße Zusammensetzung vorteilhafterweise auch noch mindestens einen Elektrolyten, vorzugsweise eine Mischung verschiedener Elektrolyte, enthalten. Dies ist besonders vorteilhaft, da auf diese Weise trotz der abführenden Wirkung der erfindungsgemäßen Zusammensetzung der Elektrolythaushalt im Gleichgewicht gehalten wird.

Die Menge an eingesetzten Elektrolyten kann in weiten Bereichen variieren; im allgemeinen liegt sie im Bereich von 0,5 bis 10 Gew.-%, vorzugsweise 1 bis 5 Gew.-%, besonders bevorzugt 1,5 bis 4 Gew.-%, ganz besonders bevorzugt 2 bis 3,5 Gew.-%, bezogen auf die erfindungsgemäße Zusammensetzung. Jedoch kann es im Einzelfall oder anwendungsbedingt erforderlich sein, von den vorgenannten Mengen abzuweichen, ohne daß der Rahmen der vorliegenden Erfindung verlassen ist. Die vorgenannten Mengenangaben bezeichnen im Falle einer Mischung verschiedener Elektrolyte die Summe aller in der erfindungsgemäßen Zusammensetzung vorhandener Elektrolyten.

Die gegebenenfalls eingesetzten Elektrolyte können insbesondere ausgewählt sein aus organischen und/oder anorganischen Alkali- und/oder Erdalkalisalzen sowie deren Mischungen. Vorzugsweise enthält die erfindungsgemäße Zusammensetzung eine Mischung sowohl organischer als auch anorganischer Alkali- und/oder Erdalkalisalze.

Das organische Alkali- und/oder Erdalkalisalz ist insbesondere ein Alkali- und/oder Erdalkalisalz einer organischen Säure, insbesondere Citronensäure und/oder Weinsäure, besonders bevorzugt Citronensäure. In erfindungsgemäß besonders bevorzugter Weise handelt es sich bei dem organischen Alkali- und/oder Erdalkalisalz um ein Alkali- und/oder Erdalkalicitrat. Derartige organische Alkali- und/oder Erdalkalisalze bedingen eine verbesserte Resorption der Elektrolyte infolge der organisch basierten Mineralstoffe und führen zudem zu einer gewünschten Alkalisierung im Magen/Darm-Trakt. Auf diese Weise kann der Elektrolythaushalt in besonderem Maße ausgeglichen werden.

Was das anorganische Alkali- und/oder Erdalkalisalz anbelangt, so handelt es sich hierbei insbesondere um ein anorganisches Chlorid, vorzugsweise Natrium- und/oder Kaliumchlorid.

Durch die erfindungsgemäß bevorzugt eingesetzte Mischung anorganischer und organischer Alkali- und/oder Erdalkalisalze wird eine besonders gute Resorption erreicht und damit einem Elektrolytverlust infolge der abführenden Wirkung der erfindungsgemäßen Zusammensetzung in effizienter Weise vorgebeugt.

Weiterhin kann es vorgesehen sein, daß die erfindungsgemäße Zusammensetzung außerdem Folsäure enthält, insbesondere in Mengen von 0,1 bis 10 Gew.-%, vorzugsweise 0,5 bis 5 Gew.-%, besonders bevorzugt 1 bis 4 Gew.-%, bezogen auf die Zusammensetzung nach der vorliegenden Erfindung. Die Folsäure hat im Rahmen der erfindungsgemäßen Zusammensetzung insbesondere die Aufgabe, regenerative Gewebe, insbesondere das Darmepithelgewebe, anzuregen.

Für eine Verbesserung der Löslichkeit bzw. Suspendierbarkeit der erfindungsgemäßen Zusammensetzung in Flüssigkeiten, insbesondere Wasser, ist es vorteilhaft, der erfindungsgemäßen Zusammensetzung außerdem bei Kontakt mit diesen Flüssigkeiten, insbesondere mit Wasser, Gas, vorzugsweise Kohlendioxid, entwickelnde Hilfsstoffe zuzusetzen, vorzugsweise anorganische Carbonate und/oder Hydrogencarbonate, im allgemeinen in Kombination mit organischen Säuren, insbesondere Citronensäure und/oder Weinsäure. Mit anderen Worten kann der erfindungsgemäßen Zusammensetzung ein sogenannter Brausesatz zugegeben werden, so daß sich die Löslichkeit bzw. Suspendierbarkeit in Wasser verbessert und die erfindungsgemäße Zusammensetzung besser peroral appliziert werden kann. Derartige Hilfsstoffe werden insbesondere in Mengen von 0,1 bis 20 Gew.-%, vorzugsweise 0,5 bis 10 Gew.-%, besonders bevorzugt 1 bis 5 Gew.-%, bezogen auf die erfindungsgemäße Zusammensetzung, zugesetzt. Dies ist dem Fachmann geläufig.

Gemäß einer erfindungsgemäß besonders bevorzugten Ausführungsform ist die Zusammensetzung nach der vorliegenden Erfindung durch die folgende Rezeptur gekennzeichnet, wobei alle nachstehend genannten Mengenangaben jeweils auf die erfindungsgemäße Zusammensetzung bezogen sind und die Summe aller Bestandteile vom Fachmann selbstverständlich derart zu kombinieren sind, daß sie in der Summe zu 100 Gew.-% führen, wobei die Rezeptur enthält:
- mindestens ein Polyethylenglykol mit einem mittleren Molekulargewicht von etwa 3.350 g/mol und/oder etwa 4.000 g/mol, vorzugsweise ein Macrogol, insbesondere in Mengen von 10 bis 99,5 Gew.-%, insbesondere 40 bis 99 Gew.-%, vorzugsweise 60 bis 99 Gew.-%, besonders bevorzugt 80 bis 98 Gew.-%, ganz besonders bevorzugt 90 bis 97 Gew.-%;
- mindestens einen Ballaststoff und/oder mindestens ein Präbiotikum, vorzugsweise Inulin, insbesondere in Mengen von 0,5 bis 30 Gew.-%, insbesondere 0,6 bis 10 Gew.-%, vorzugsweise 0,7 bis 5 Gew.-%, besonders bevorzugt 0,8 bis 3 Gew.-%, ganz besonders bevorzugt 1 bis 2 Gew.-%;
- mindestens einen Elektrolyten, vorzugsweise eine Mischung verschiedener Elektrolyte, insbesondere eine Mischung von Alkalichlorid(en) und Alkali- und/oder Erdalkalisalzen organischer Säuren, bevorzugt Citraten, insbesondere in Mengen von insgesamt 0,5 bis 10 Gew.-%, vorzugsweise 1 bis 5 Gew.-%, besonders bevorzugt 1,5 bis 4 Gew.-%, ganz besonders bevorzugt 2 bis 3,5 Gew.-%.

Neben den vorgenannten Wirk- und/oder Inhaltsstoffen kann die erfindungsgemäße Zusammensetzung außerdem übliche pharmazeutische Zusatz- und/oder Hilfsstoffe enthalten. Diese können insbesondere ausgewählt sein aus der Gruppe von Füll-, Streck-, Binde-, Netz-, Stabilisierungs-, Färbe-, Puffer-, Riech-, Geschmacks- und/oder Konservierungsstoffen.

Im allgemeinen liegt die erfindungsgemäße Zusammensetzung in fester Form, vorzugsweise in Form eines Pulvers oder Granulats, vor und ist für die perorale Applikation nach Anrühren mit Wasser bestimmt.

Vorteilhafterweise liegt die erfindungsgemäße Zusammensetzung dosierfertig für die einmalige Applikation in Portionsbeuteln verpackt vor, insbesondere in Mengen von 5 bis 50 g, vorzugsweise 10 bis 40 g, je Portionsbeutel. Für die perorale Applikation kann dann die vorgenannte Menge der erfindungsgemäßen Zusammensetzung mit ausreichend Flüssigkeit (z. B. ca. 100 bis 200 ml Wasser) zu einer Lösung bzw. Suspension zubereitet und peroral appliziert werden.

Weiterer Gegenstand der vorliegenden Erfindung - gemäß einem **zweiten** Aspekt der vorliegenden Erfindung - ist die Verwendung der Zusammensetzung nach der vorliegenden Erfindung als Laxativum bzw. Abführmittel (Laxans).

Für die Zwecke der erfindungsgemäßen Verwendung wird die erfindungsgemäße Zusammensetzung, wie zuvor beschrieben, peroral appliziert, im allgemeinen als wäßrige Lösung oder Suspension.

Im allgemeinen wird die Zusammensetzung in Tagesdosen von 1 bis 100 g, insbesondere 5 bis 50 g, vorzugsweise 10 bis 40 g, der Zusammensetzung appliziert.

Wie zuvor beschrieben, kann die erfindungsgemäße Zusammensetzung zur symptomatischen Behandlung bei Verstopfung (Obstipation) angewendet werden. Es handelt sich um ein Abführmitteln, welches aufgrund seiner einzigartigen Zusammensetzung nicht nur besonders zuverlässig und schonend wirkt, sondern aufgrund des Vorhandenseins der Elektrolyte zusätzlich den Mineralstoffhaushalt ausgleicht, insbesondere durch die Kombination anorganischer und organischer Elektrolyte, sowie durch das Vorhandensein der Ballaststoffe und/oder Präbiotika die Darmflora stärkt bzw. regeneriert und somit die Verdauung bzw. Darmtätigkeit eigenständig anregt. Im allgemeinen enthält die erfindungsgemäße Zusammensetzung neben dem Polyol und dem Ballaststoff und/oder Präbiotikum vorzugsweise eine Mischung anorganischer und organischer Mineralstoffe.

Das Polyol dient als Trägersubstanz für die Flüssigkeit (Wasser), mit der die Trinklösung oder Trinksuspension eingenommen wird. Zusätzlich bindet es Wasser im Darm, so daß der verhärtete Stuhl durch die Flüssigkeit aufgeweicht und das Stuhlvolumen erhöht wird. Dadurch wird auf natürliche Weise die Darmbewegung (Darmperistaltik) ausgelöst. Die mitgeführten Elektrolyte sorgen dafür, daß der Mineralhaushalt im Gleichgewicht bleibt. Darüber hinaus stärkt das Vorhandensein der Ballaststoffe und/oder der Präbiotika, insbesondere von Inulin, die Darmflora, die wichtige Bakterien für die Funktion und Gesunderhaltung des Darms enthält. Auf diese Weise wird durch die erfindungsgemäße Zusammensetzung die Darmtätigkeit dreifach positiv unterstützt, und dies ohne Gewöhnungseffekt und besonders zuverlässig und schonend.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne daß er dabei den Rahmen der vorliegenden Erfindung verläßt.

Die vorliegende Erfindung wird anhand der nachfolgenden Ausführungsbeispiele veranschaulicht.

### AUSFÜHRUNGSBEISPIELE:

Eine erste erfindungsgemäße Zusammensetzung (Zusammensetzung I) wird mit den folgenden Bestandteilen (ohne Angabe von üblichen Hilfs- und Zusatzstoffen) formuliert, wobei eine mit Wasser einzunehmende Applikationseinheit jeweils ca. 14 g enthält, welche zur Einnahme in jeweils ca. 100 bis 200 ml Flüssigkeit aufgelöst bzw. suspendiert werden kann: 95 Gew.-% Polyethylenglykol mit einem mittleren Molekulargewicht von ca. 4.000 g/mol (Macrogol 4000), 2 Gew.-% Inulin und 3 Gew.-% Elektrolyte (Magnesiumcitrat, Calciumcitrat und Kaliumchlorid in einem Gewichtsverhältnis von ca. 1:2: 0,24).

In gleicher Weise wird eine weitere erfindungsgemäße Zusammensetzung (Zusammensetzung II) formuliert, bei der jedoch das Präbiotikum Inulin vollständig gegen dieselbe Menge an Lactulose ausgetauscht ist.

Weiterhin wird eine nichterfindungsgemäße Vergleichszusammensetzung formuliert, bei welcher der Anteil an Präbiotikum durch Macrogol 4000 ersetzt ist, d. h. die nichterfindungsgemäße Vergleichszusammensetzung ist vollständig ohne Präbiotikum formuliert.

Jeweils vier Probanden (d. h. also insgesamt zwölf Probanden) im Alter zwischen 50 und 75 Jahren werden mit den vorgenannten Zusammensetzungen durch dreimalige Gabe pro Tag behandelt.

Dabei werden über einen Zeitraum von jeweils einem Monat mit der inulinhaltigen erfindungsgemäßen Zusammensetzung (Zusammensetzung I) die besten Ergebnisse beobachtet. Die betreffenden Patienten können bereits nach zweitätiger Einnahme wieder regelmäßig entleeren.

Bei der erfindungsgemäßen Zusammensetzung II tritt dieser Effekt erst nach etwa vier bis fünf Tagen auf und ist nicht so nachhaltig wie im Fall der inulinhaltigen Zusammensetzung II, aber immer noch sehr zufriedenstellend.

Im Fall der mit der Vergleichszusammensetzung behandelten Patienten dagegen können die Obstipationen nicht in ausreichendem Maße ausgeräumt werden; insbesondere fehlt es an einer Stimulation der Darmtätigkeit. Folglich wird bei diesen Patienten nach drei Wochen ein Therapiewechsel zu der erfindungsgemäßen Zusammensetzung I vorgenommen, woraufhin in allen Fällen ein ausreichender abführender Effekt nachhaltig eintritt.

Die Ergebnisse belegen die vorteilhaften Eigenschaften einer Kombination eines Polyols mit einem Präbiotikum im Vergleich zum Polyol allein. Da mit allen Zusammensetzungen eine ausreichende Menge an bioverfügbaren Elektrolyten bereitgestellt wird, wird der Elektrolythaushalt im Gleichgewicht gehalten. Die besten Effekte werden mit Inulin als Präbiotikum erhalten.

## Patentansprüche

1. Zusammensetzung, insbesondere pharmazeutische Zusammensetzung, enthaltend in Kombination
- mindestens ein Polyol in Mengen von 10 bis 99,5 Gew.-%,
- mindestens einen Ballaststoff und/oder mindestens ein Präbiotikum in Mengen von 0,5 bis 30 Gew.-% und
- mindestens einen Elektrolyten in Mengen von 0,5 bis 10 Gew.-%,
wobei alle vorgenannten Mengenangaben jeweils auf die Zusammensetzung bezogen sind.

2. Zusammensetzung nach Anspruch 1, enthaltend Polyol(e), bezogen auf die Zusammensetzung, in Mengen von 40 bis 99 Gew.-%, vorzugsweise 60 bis 99 Gew.-%, besonders bevorzugt 80 bis 98 Gew.-%, ganz besonders bevorzugt 90 bis 97 Gew.-%, und/oder enthaltend Ballaststoff(e) und/oder Präbiotikum, bezogen auf die Zusammensetzung, in Mengen von 0,6 bis 10 Gew.-%, vorzugsweise 0,7 bis 5 Gew.-%, besonders bevorzugt 0,8 bis 3 Gew.-%, ganz besonders bevorzugt 1 bis 2 Gew.-%.

3. Zusammensetzung nach Anspruch 1 oder 2, wobei das Polyol osmotisch wirksam ist, insbesondere osmotisch Wasser bindet, und/oder wobei das Polyol in einer Flüssigkeit, insbesondere Wasser, löslich und/oder quellfähig ist und/oder wobei das Polyol zumindest im wesentlichen nichtresorbierbar ist und/oder wobei das Polyol ein Polyalkylenglykol, vorzugsweise ein Polyethylenglykol, ist und/oder wobei das Polyol ein mittleres Molekulargewicht im Bereich von 1.000 bis 10.000 g/mol, insbesondere 2.000 bis 8.000 g/mol, vorzugsweise 2.500 bis 5.500 g/mol, bevorzugt 3.000 bis 5.000 g/mol, besonders bevorzugt 3.200 bis 4.200 g/mol, aufweist.

4. Zusammensetzung nach einem oder mehreren der vorangehenden Ansprüche, wobei das Polyol ein Polyethylenglykol mit einem mittleren Molekulargewicht im Bereich von 2.500 bis 5.500 g/mol, bevorzugt 3.000 bis 5.000 g/mol, ganz besonders bevorzugt 3.200 bis 4.200 g/mol, ist und/oder wobei das Polyol ein Polyethylenglykol mit einem mittleren Molekulargewicht von etwa 3.350 g/mol oder etwa 4.000 g/mol oder eine Mischung eines Polyethylenglykols mit einem mittleren Molekulargewicht von etwa 3.350 g/mol und eines Polyethylenglykols mit einem mittleren Molekulargewicht etwa 4.000 g/mol, vorzugsweise vom Typ Macrogol 3350 und/oder Macrogol 4000, ist.

5. Zusammensetzung nach einem oder mehreren der vorangehenden Ansprüche, wobei der Ballaststoff und/oder das Präbiotikum ausgewählt ist aus der Gruppe von Cellulose, Lignin, Pentosanen, Keratinen, Agar-Agar, löslichen Ballaststoffen, wie Fructo- oder Galactooligosacchariden, vorzugsweise Inulin, sowie deren Derivaten und Mischungen und/ oder wobei der Ballaststoff und/oder das Präbiotikum in Form von Inulin, Flohsamen, Leinsamen, Guar und/oder Lactulose zugesetzt ist und/ oder wobei der Ballaststoff und/oder das Präbiotikum Inulin ist.

6. Zusammensetzung nach einem oder mehreren der vorangehenden Ansprüche, enthaltend eine Mischung verschiedener Elektrolyte, insbesondere in Mengen von 1 bis 5 Gew.-%, besonders bevorzugt 1,5 bis 4 Gew.-%, ganz besonders bevorzugt 2 bis 3,5 Gew.-%, bezogen auf die Zusammensetzung, insbesondere wobei der oder die Elektrolyte ausgewählt sind aus organischen und/oder anorganischen Alkali- und/oder Erdalkalisalzen sowie deren Mischungen, vorzugsweise einer Mischung organischer und anorganischer Alkali- und/oder Erdalkalisalzen.

7. Zusammensetzung nach Anspruch 6, wobei das organische Alkali- und/oder Erdalkalisalz ein Alkali- und/oder Erdalkalisalz einer organischen Säure, insbesondere Citronensäure und/oder Weinsäure, besonders bevorzugt Citronensäure, ist und in ganz besonders bevorzugter Weise ein Alkali- und/oder Erdalkalicitrat ist und/oder wobei das anorganische Alkali- und/oder Erdalkalisalz ein Chlorid, insbesondere Natrium- und/oder Kaliumchlorid, ist.

8. Zusammensetzung nach einem oder mehreren der vorangehenden Ansprüche, enthaltend außerdem Folsäure, insbesondere in Mengen von 0,1 bis 10 Gew.-%, vorzugsweise 0,5 bis 5 Gew.-%, besonders bevorzugt 1 bis 4 Gew.-%, bezogen auf die Zusammensetzung.

9. Zusammensetzung nach einem oder mehreren der vorangehenden Ansprüche, enthaltend außerdem mindestens einen bei Kontakt mit Wasser Gas, insbesondere Kohlendioxid, entwickelnden Hilfsstoff, vorzugsweise ein anorganisches Carbonat und/oder Hydrogencarbonat, gegebenenfalls in Kombination mit organischen Säuren, wie insbesondere Citronensäure und/oder Weinsäure, insbesondere in Mengen von 0,1 bis 20 Gew.-%, vorzugsweise 0,5 bis 10 Gew.-%, besonders bevorzugt 1 bis 5 Gew.-%, bezogen auf die Zusammensetzung.

10. Zusammensetzung, insbesondere nach einem oder mehreren der vorangehenden Ansprüche, **gekennzeichnet durch** die folgende Rezeptur, enthaltend:
- mindestens ein Polyethylenglykol mit einem mittleren Molekulargewicht von etwa 3.350 g/mol und/oder etwa 4.000 g/mol, vorzugsweise ein Macrogol, insbesondere in Mengen von 10 bis 99,5 Gew.-%, insbesondere 40 bis 99 Gew.-%, vorzugsweise 60 bis 99 Gew.-%, besonders bevorzugt 80 bis 98 Gew.-%, ganz besonders bevorzugt 90 bis 97 Gew.-%;
- mindestens einen Ballaststoff und/oder mindestens ein Präbiotikum, vorzugsweise Inulin, insbesondere in Mengen von 0,5 bis 30 Gew.-%, insbesondere 0,6 bis 10 Gew.-%, vorzugsweise 0,7 bis 5 Gew.-%, besonders bevorzugt 0,8 bis 3 Gew.-%, ganz besonders bevorzugt 1 bis 2 Gew.-%;
- mindestens einen Elektrolyten, vorzugsweise eine Mischung verschiedener Elektrolyte, insbesondere eine Mischung von Alkalichlorid(en) und Alkali- und/oder Erdalkalisalzen organischer Säuren, bevorzugt Citraten, insbesondere in Mengen von insgesamt 0,5 bis 10 Gew.-%, vorzugsweise 1 bis 5 Gew.-%, besonders bevorzugt 1,5 bis 4 Gew.-%, ganz besonders bevorzugt 2 bis 3,5 Gew.-%;
wobei alle vorgenannten Mengenangaben jeweils auf die Zusammensetzung bezogen sind.

11. Zusammensetzung nach einem oder mehreren der vorangehenden Ansprüche, enthaltend außerdem übliche pharmazeutische Zusatz- und/oder Hilfsstoffe, insbesondere aus der Gruppe von Füll-, Streck-, Binde-, Netz-, Stabilisierungs-, Färbe-, Puffer-, Riech-, Geschmacks- und/oder Konservierungsstoffen.

12. Zusammensetzung nach einem oder mehreren der vorangehenden Ansprüche, wobei die Zusammensetzung in fester Form, vorzugsweise in Form eines Pulvers oder Granulats, vorliegt.

13. Zusammensetzung nach einem oder mehreren der vorangehenden Ansprüche für die perorale Applikation nach Anrühren mit Wasser und/ oder zur Verwendung als Laxativum (Abführmittel).

14. Zusammensetzung nach einem oder mehreren der vorangehenden Ansprüche, wobei die Zusammensetzung dosierfertig in Portionsbeuteln verpackt vorliegt, insbesondere in Mengen von 5 bis 50 g, vorzugsweise 10 bis 40 g, je Portionsbeutel.

15. Verwendung einer Zusammensetzung nach einem oder mehreren der vorangehenden Ansprüche zur Herstellung eines als Laxativum (Abführmittel) wirkenden Arzneimittels, insbesondere wobei die Zusammensetzung peroral appliziert wird, vorzugsweise als wäßrige Lösung oder Suspension, und/oder insbesondere wobei die Zusammensetzung in Tagesdosen von 1 bis 100 g, insbesondere 5 bis 50 g, vorzugsweise 10 bis 40 g, der Zusammensetzung appliziert wird.

## Claims

1. Composition, in particular a pharmaceutical composition, comprising in combination:
- at least one polyol in amounts of from 10 to 99.5 wt.-%,
- at least one fiber and/or at least one prebiotic in amounts of from 0.5 to 30 wt.-%, and
- at least one electrolyte in amounts of from 0.5 to 10 wt.-%,
wherein all the abovementioned quantities are respectively based on the composition.

2. Composition according to claim 1, comprising polyol(s), based on the composition, in amounts of from 40 to 99 wt.-%, preferably from 60 to 99 wt.-%, particularly preferably from 80 to 98 wt.-%, very particularly preferably from 90 to 97 wt.-%, and/or containing fiber(s) and/or prebiotics based on the composition, in amounts of from 0.6 to 10 wt.-%, preferably from 0.7 to 5 wt.-%, particularly preferably from 0.8 to 3 wt.-%, very particularly preferably from 1 to 2 wt.-%.

3. Composition according to claim 1 or 2, wherein the polyol is osmotically active, in particular osmotically binding water, and/or wherein the polyol is soluble and/or swellable in a liquid, in particular water, and/or wherein the polyol is at least substantially non-resorbable, and/or wherein the polyol is a polyalkylene glycol, preferably a polyethylene glycol, and/or wherein the polyol has an average molecular weight in the range from 1,000 to 10,000 g/mol, in particular 2,000 to 8,000 g/mol, preferably 2,500 to 5,500 g/mol, more preferably 3,000 to 5,000 g/mol, particularly preferably 3,200 to 4,200 g/mol.

4. Composition according to one or more of the preceding claims, wherein the polyol is a polyethylene glycol having an average molecular weight in the range from 2,500 to 5,500 g/mol, preferably from 3,000 to 5,000 g/mol, very particularly preferably from 3,200 to 4,200 g/mol, and/or wherein the polyol is a polyethylene glycol having an average molecular weight of about 3,350 g/mol or about 4,000 g/mol or a mixture of a polyethylene glycol having an average molecular weight of about 3,350 g/mol and a polyethylene glycol having an average molecular weight of about 4,000 g/mol, preferably of the Macrogol 3350 and/or Macrogol 4000 type.

5. Composition according to one or more of the preceding claims, wherein the fiber and/or the prebiotic is selected from the group consisting of cellulose, lignin, pentosans, keratins, agar agar, soluble dietary fibers such as fructo- or galactooligosaccharides, preferably inulin, as well as their derivatives and mixtures, and/or wherein the fiber and/or the prebiotic is inulin, flea seed, linseed, guar and/or lactulose, and/or wherein the dietary fiber and/or the prebiotic is inulin.

6. Composition according to one or more of the preceding claims, comprising a mixture of different electrolytes, in particular in amounts of 1 to 5 wt.-%, particularly preferably 1.5 to 4 wt.-%, very particularly preferably 2 to 3.5 wt.-%, based on the composition, in particular wherein the electrolyte(s) is/are selected from organic and/or inorganic alkali and/or earth metal salts as well as their mixtures, preferably a mixture of organic and inorganic alkali and/or earth metal salts.

7. Composition according to claim 6, wherein the organic alkali and/or alkaline earth metal salt is an alkali and/or alkaline earth metal salt of an organic acid, especially citric acid and/or tartaric acid, particularly preferably citric acid, and most preferably an alkali and/or alkaline earth citrate, and/or wherein the inorganic alkali and/or alkaline earth metal salt is a chloride, in particular sodium chloride and/or potassium chloride.

8. Composition according to one or more of the preceding claims, further comprising folic acid, in particular in amounts of 0.1 to 10 wt.-%, preferably 0.5 to 5 wt.-%, particularly preferably 1 to 4 wt.-%, based on the composition.

9. Composition according to one or more of the preceding claims, further comprising at least one excipient which develops on contact with water gas, in particular carbon dioxide, preferably an inorganic carbonate and/or hydrocarbonate, optionally in combination with organic acids such as, in particular, citric acid and/or tartaric acid, In particular in amounts of from 0.1 to 20 wt.-%, preferably from 0.5 to 10 wt.-%, particularly preferably from 1 to 5 wt.-%, based on the composition.

10. Composition, in particular according to one or more of the preceding claims, **characterized by** the following formulation, comprising:
- at least one polyethylene glycol having an average molecular weight of about 3,350 g/mol and/or about 4,000 g/mol, preferably a macrogol, in particular in amounts from 10 to 99.5 wt.-%, in particular from 40 to 99 wt.-%, preferably 60 to 99 wt.-%, particularly preferably 80 to 98 wt.-%, very particularly preferably 90 to 97 wt.-%;
- at least one fiber and/or at least one prebiotic, preferably inulin, in particular in amounts from 0.5 to 30 wt.-%, in particular from 0.6 to 10 wt.-%, preferably from 0.7 to 5 wt.-%, particularly preferably from 0.8 to 3 wt.-%, very particularly preferably from 1 to 2 wt.-%;
- at least one electrolyte, preferably a mixture of different electrolytes, in particular a mixture of alkali metal chlorides and alkali metal and/or alkaline earth metal salts of organic acids, preferably citrates, in particular in amounts of 0.5 to 10 wt.-%, preferably 1 to 5 wt.-%, particularly preferably 1.5 to 4 wt.-%, very particularly preferably 2 to 3.5 wt.-%;
wherein all the abovementioned quantities are respectively based on the composition.

11. Composition according to one or more of the preceding claims, further comprising conventional pharmaceutical additives and/or excipients, in particular from the group of filling, stretching, binding, net, stabilizing, dyeing, buffering, odorous, flavoring and/or preservative substances.

12. Composition according to one or more of the preceding claims, wherein the composition is in solid form, preferably in the form of a powder or granules.

13. Composition according to one or more of the preceding claims for peroral administration after stirring with water and/or for use as a laxative.

14. Composition according to one or more of the preceding claims, wherein the composition is packaged ready for dosage in sachets, in particular in amounts of 5 to 50 g, preferably 10 to 40 g, per portion sachet.

15. Use of a composition according to one or more of the preceding claims for the manufacture of a medicament acting as a laxative, in particular wherein the composition is administered perorally, preferably as an aqueous solution or suspension, wherein the composition is applied in daily doses of from 1 to 100 g, in particular from 5 to 50 g, preferably from 10 to 40 g, of the composition.

## Revendications

1. Composition, en particulier composition pharmaceutique, contenant en combinaison
- au moins un polyol en des quantités de 10 à 99,5 % en poids,
- au moins une fibre alimentaire et/ou au moins un prébiotique en des quantités de 0,5 à 30 % en poids, et
- au moins un électrolyte en des quantités de 0,5 à 10 % en poids,
dans laquelle toutes les indications de quantités ci-dessus sont chacune rapportées à la composition.

2. Composition selon la revendication 1, contenant un ou plusieurs polyols, en des quantités, rapportées à la composition, de 40 à 99 % en poids, de préférence de 60 à 99 % en poids, d'une manière particulièrement préférée de 80 à 98 % en poids, d'une manière tout particulièrement préférée de 90 à 97 % en poids, et/ou contenant une ou plusieurs fibres alimentaires et/ou un prébiotique, en des quantités, rapportées à la composition, de 0,6 à 10 % en poids, de préférence de 0,7 à 5 % en poids, d'une manière particulièrement préférée de 0,8 à 3 % en poids, d'une manière tout particulièrement préférée de 1 à 2 % en poids.

3. Composition selon la revendication 1 ou 2, dans laquelle le polyol a une activité osmotique, en particulier lie l'eau par liaison osmotique, et/ou dans laquelle le polyol est soluble et/ou peut gonfler dans un liquide, en particulier l'eau, et/ou dans laquelle le polyol est au moins pour l'essentiel non résorbable, et/ou dans laquelle le polyol est un polyalkylèneglycol, de préférence un polyéthylèneglycol et/ou dans laquelle le polyol présente une masse moléculaire moyenne comprise dans la plage de 1000 à 10 000 g/mol, en particulier de 2000 à 8000 g/mol, de préférence de 2500 à 5500 g/mol, préférentiellement de 3000 à 5000 g/mol, d'une manière particulièrement préférée de 3200 à 4200 g/mol.

4. Composition selon l'une ou plusieurs des revendications précédentes, dans laquelle le polyol est un polyéthylèneglycol ayant une masse moléculaire moyenne comprise dans la plage de 2500 à 5500 g/mol, préférentiellement de 3000 à 5000 g/mol, d'une manière tout particulièrement préférée de 3200 à 4200 g/mol, et/ou dans laquelle le polyol est un polyéthylèneglycol ayant une masse moléculaire moyenne d'environ 3350 g/mol ou d'environ 4000 g/mol, ou un mélange d'un polyéthylèneglycol ayant une masse moléculaire moyenne d'environ 3350 g/mol et d'un polyéthylèneglycol ayant une masse moléculaire moyenne d'environ 4000 g/mol, de préférence des types Macrogol 3350 et/ou Macrogol 4000.

5. Composition selon l'une ou plusieurs des revendications précédentes, dans laquelle la fibre alimentaire et/ou le prébiotique sont choisis dans le groupe consistant en la cellulose, la lignine, les pentosanes, les kératines, l'agar-agar, les fibres solubles telles que les fructo- ou les galactooligosaccharies, de préférence l'inuline, ainsi que les dérivés et mélanges de ceux-ci, et/ou dans laquelle la fibre alimentaire et/ou le prébiotique sont ajoutés sous forme d'inuline, de graines de plantain psyllium, de graines de lin, de gomme de guar et/ou de laculose, et/ou dans laquelle la fibre alimentaire et/ou le prébiotique sont l'inuline.

6. Composition selon l'une ou plusieurs des revendications précédentes, contenant un mélange de différents électrolytes, en particulier en des quantités de 1 à 5 % en poids, d'une manière particulièrement préférée de 1,5 à 4 % en poids, d'une manière tout particulièrement préférée de 2 à 3,5 % en poids, par rapport à la composition, en particulier dans laquelle le ou les électrolytes sont choisis parmi les sels organiques et/ou inorganiques de métaux alcalins et/ou alcalino-terreux, ainsi que les mélanges de ceux-ci, de préférence un mélange de sels organiques et inorganiques de métaux alcalins et/ou alcalino-terreux.

7. Composition selon la revendication 6, dans laquelle le sel organique d'un métal alcalin et/ou alcalino-terreux est un sel d'un métal alcalin et/ou alcalino-terreux d'un acide organique, en particulier l'acide citrique et/ou l'acide tartrique, d'une manière particulièrement préférée l'acide citrique, et, d'une manière tout particulièrement préférée, est un citrate d'un métal alcalin et/ou alcalino-terreux, et/ou dans laquelle le sel inorganique d'un métal alcalin et/ou alcalino-terreux est un chlorure, en particulier le chlorure de sodium et/ou le chlorure de potassium.

8. Composition selon l'une ou plusieurs des revendications précédentes, contenant en outre de l'acide folique, en particulier en des quantités de 0,1 à 10 % en poids, de préférence de 0,5 à 5 % en poids, d'une manière particulièrement préférée de 1 à 4 % en poids, par rapport à la composition.

9. Composition selon l'une ou plusieurs des revendications précédentes, contenant en outre au moins un adjuvant, qui par contact avec de l'eau dégage un gaz, en particulier le dioxyde de carbone, de préférence un carbonate et/ou un hydrogénocarbonate inorganique, éventuellement en combinaison avec des acides organiques tels en particulier que l'acide citrique et/ou l'acide tartrique, en particulier en des quantités de 0,1 à 20 % en poids, de préférence de 0,5 à 10 % en poids, d'une manière particulièrement préférée de 1 à 5 % en poids, par rapport à la composition.

10. Composition, en particulier selon l'une ou plusieurs des revendications précédentes, **caractérisée par** la formulation suivante, contenant :
- au moins un polyéthylèneglycol ayant une masse moléculaire moyenne d'environ 335.0 g/mol et/ou d'environ 4000 g/mol, de préférence un Macrogol, en particulier en des quantités de 10 à 99,5 % en poids, en particulier de 40 à 99 % en poids, de préférence de 60 à 99 % en poids, d'une manière particulièrement préférée de 80 à 98 % en poids, d'une manière tout particulièrement préférée de 90 à 97 % en poids ;
- au moins une fibre alimentaire et/ou un prébiotique, de préférence l'inuline, en particulier en des quantités de 0,5 à 30 % en poids, en particulier de 0,6 à 10 % en poids, de préférence de 0,7 à 5 % en poids, d'une manière particulièrement préférée de 0,8 à 3 % en poids, d'une manière tout particulièrement préférée de 1 à 2 % en poids ;
- au moins un électrolyte, de préférence un mélange de différents électrolytes, en particulier un mélange d'un ou plusieurs chlorures de métaux alcalins et de sels de métaux alcalins et/ou alcalino-terreux d'acides inorganiques, de préférence les citrates, en particulier en des quantités totales de 0,5 à 10 % en poids, de préférence de 1 à 5 % en poids, d'une manière particulièrement préférée de 1,5 à 4 % en poids, d'une manière tout particulièrement préférée de 2 à 3,5 % en poids ;
toutes les indications de quantités ci-dessus étant chacune rapportées à la composition.

11. Composition selon l'une ou plusieurs des revendications précédentes, contenant en outre des additifs et/ou adjuvants pharmaceutiques usuels, en particulier du groupe consistant en les charges, les diluants, les liants, les mouillants, les stabilisants, les colorants, les substances tampons, les aromatisants, les agents de sapidité et/ou les conservateurs.

12. Composition selon l'une ou plusieurs des revendications précédentes, la composition se présentant sous forme solide, de préférence sous forme d'une poudre ou d'un granulé.

13. Composition selon l'une ou plusieurs des revendications précédentes, pour application par voie orale après délayage avec de l'eau et/ou pour une utilisation en tant que laxatif (purgatif).

14. Composition selon l'une ou plusieurs des revendications précédentes, la composition se présentant conditionnée, prête au dosage, en sachets-portions, en particulier en des quantités de 5 à 50 g, de préférence de 10 à 40 g par sachet-portion.

15. Utilisation d'une composition selon l'une ou plusieurs des revendications précédentes pour fabriquer un médicament à effet laxatif (purgatif), en particulier pour laquelle la composition est appliquée par voie orale, de préférence sous forme d'une solution ou d'une suspension aqueuse, et/ou en particulier dans laquelle la composition est administrée à des doses journalières de 1 à 100 g, en particulier de 5 à 50 g, de préférence de 10 à 40 g, de la composition.
